# EUROPEAN PATENT SPECIFICATION

(11) **EP 1 388 588 B1**
(45) Date of publication and mention of the grant of the patent: **17.09.2008**
(21) Application number: 03017105.2
(22) Date of filing: 28.07.2003
(51) Int. Cl.: C12N 15/10

(54) **Nucleic acid-separating method and nucleic acid-extracting reagent**
Methode zur Separierung von Nukleinsäuren und dafür verwendbare Reagenz
Méthode de séparation d'acide nucléiques et solutions pour les extraire

(30) Priority: 29.07.2002 JP 2002220099
(43) Date of publication of application: 11.02.2004
(73) Proprietor: JSR Corporation, Tokyo 104-0045 (JP)
(72) Inventor: Fan, Kejun, Tokyo (JP)
(74) Representative: TBK-Patent

(56) References cited:
- WO-A-00/40697
- WO-A-01/42456
- WO-A-91/12079
- WO-A-92/13963
- WO-A-96/18731
- WO-A-99/58664
- US-A- 6 117 398
- QIAGEN: "Qiagen Genomic DNA Handbook" [Online] August 2001 (2001-08) , QIAGEN XP002255737 Retrieved from the Internet: <URL: http://www1.qiagen.com/literature/handbook s/PDF/GenomicDNAStabilizationAndPurificati on/FromAnimalAndPlantIssues/GENO/1018833_H B_GENO_082001.pdf> [retrieved on 2003-09-25] pages 30-31, 55-57

## Description

### FIELD OF THE INVENTION

The present invention relates to a method for separating nucleic acids from a sample containing the nucleic acids and to a reagent to be used for carrying out the method. Especially, it relates to a method for purifying nucleic acids wherein objective nucleic acids are separated through solid-liquid separation by deposition of the nucleic acids onto a solid-phase carrier, via enzymatic treatment of a cell lysis solution, from a sample containing nucleated cells and the like, and to a kit for carrying out the method.

### BACKGROUND OF THE INVENTION

Recently, the application of nucleic acid analysis has been expanded to various fields such as science research, medical use, and industry and it has been desired to develop a method capable of extracting and purifying a large amount of highly pure nucleic acids by efficient operations.

As a method for obtaining nucleic acids from a sample containing the nucleic acids, a phenol-chloroform extraction method has been utilized for a long time. In this method, analyte components hardly soluble in water, such as proteins and lipids are denatured, dissolved or precipitated using phenol-chloroform. The method utilizes the difference in solubility that a strongly hydrophilic nucleic acid having an extremely high charge dissolves in an aqueous phase. This method enables the extraction of highly pure nucleic acids but is not suitable for the extraction of nucleic acids from a large amount of analyte owing to the limitations of working conditions since phenol and chloroform are toxic.

As an alternative method using no such toxic solvents, there is the Boom method (Boom et al., J. Clin. Microbiol. 28: 495-503 (1990)) wherein contaminants such as proteins and lipids are solubilized in an aqueous phase using a chaotropic solution and nucleic acids are adsorbed onto silica beads to recover them as a solid phase, and then the nucleic acids are again dissolved in an aqueous phase. In this method, the extracted nucleic acids are frequently short and fragmented. In particular, the method is not suitable for DNA assays such as Southern blot technique.

In addition to the methods, a reagent coprecipitating DNA which utilizes a cationic polymer has been developed but a fine tuning of the reagent is required for the separation of DNA and also centrifugation operations are unavoidable throughout the separation because the separation is a liquid-liquid separation. Therefore, the efficiency of routine treatment of a large number of analytes is low, and hence its application in industry is not advanced.

Moreover, in the method of using a chaotropic solution and a silica carrier, the capable capacity of analyte to be treated is less than 1 mL and the purity of nucleic acids recovered tends to remarkably decrease when the analyte volume increases. This is due to the adsorbability of the silica carrier used. When the amount of the silica carrier is increased, the remaining amount of the chaotropic salt in the solid-liquid separation also increases since dead volume is proportional to the amount of the carrier used. This can be coped with the increase of washing times but it results in an increase of operating period of time and complication of the operating steps, so that it is not an effective measure for solving the problem.

On the other hand, a method of precipitating nucleic acids from a nucleic acid-containing solution with an ethanol solution and rolling them up with a glass rod has been known from long ago (for example, "Kiso Seikagaku Jikken-ho (Basic Biochemical Experimental Methods) 2", ed. by Kouichi Anan, Maruzen, 1974). This procedure utilizes a factor based on the affinity between glass and nucleic acids but there is observed a contamination of polymers other than nucleic acids and hence the purity of recovered nucleic acids is low, so that its practical application is limited.

As mentioned above, it is strongly desired to develop a method capable of extracting and purifying highly pure nucleic acids conveniently and rapidly using no toxic solvent or corrosive reagent and having a possibility of automation. In consideration of such a situation, the present inventors conducted extensive studies and found the formation of a coprecipitated state of nucleic acids in a sample with fine particles induced by adding a water-soluble organic solvent in a state where nucleated cells are lysed and subjected to a certain treatment with an enzyme or the like. By utilizing this, the present inventors realized a convenient solid-liquid separation which solves the problems of the above conventional methods at once, thereby they have accomplished the invention. Based on the method according to the invention, it becomes also possible to realize the automation of the operation separating highly pure nucleic acids from a blood analyte.

WO 00/40697 A discloses methods and kits for isolation of nucleic acid molecules. Nucleic acid molecules are isolated using an integrated lysis/chromatography matrix, which may comprise one or more supports (e.g. polyolefin, scintered polyethylene, nitrocellulose, polypropylene, polycarbonate, cellulose acetate, silica, and the like).

WO 99/58664 A discloses a solid phase technique for selectively isolating nucleic acids by selectively facilitating the adsorption of a particular species of nucleic acid molecule to the functional group-coated surface of magnetically responsive paramagnetic microparticles.

WO 92/13963 A discloses a method for preparation of closed circular DNA. In the final step, plasmid DNA is recovered from a solution by ultrafiltration, precipitation, binding to a particle or surface which binds nucleic acids such as hydroxyapatite, glass milk or DEAE beads, centrifugation or isobutanol extraction.

US 6,117,398 A discloses a system for releasing and isolating nucleic acids, in which nucleic acids released from a biological compartment bind non-specifically to the surface of glass-magnetic particles.

WO 91/12079 A discloses a method recovering a biopolymer from solution which involves the use of magnetically attractable beads which do not specifically bind the polymer. The beads comprise finely divided magnetisable material encapsulated in an organic polymer.

WO 96/18731 A discloses a method of isolating nucleic acid from a sample comprising contacting the sample with a detergent and a solid support. The support may be made of glass, silica, latex or a polymer material.

### SUMMARY OF THE INVENTION

It is an object of the invention to provide a novel means for rapidly extracting only nucleic acids by converting biopolymers other than nucleic acids into low molecular weight compounds through an enzymatic treatment using no toxic solvent such as phenol or chloroform and also no corrosive reagent such as a chaotropic substance.

The invention relates to a method for separating nucleic acids from a sample containing nucleated cells as defined in claim 1.

The invention also relates to a nucleic acid-extracting reagent kit as defined in claim 6. Preferred embodiments are defined in the subclaims.

### DETAILED DESCRIPTION OF THE INVENTION

The following will describe the present method together with the means for carrying out the method.

In the present specification, "nucleic acid" means DNA and/or RNA as double-stranded (ds) form, single-stranded (ss) form or a combination thereof (partial ds or ss).

The nucleated cell that is a target of the invention is present widely in the realm of nature and the kind of animal is not limited. Specifically, blood, bone marrow, cord blood, tissues, cultured cells, urine, saliva, and the like are target analytes of the invention. Depending on the nature of these analytes, a physical operation may be applied thereto for easy release of nucleated components prior to the application of the invention. For example, with regard to an analyte collected from an organ or tissue, the analyte can be applied to the invention after the tissue structure has been crushed by means of a mixer or the like.

Moreover, the shape and amount of the analyte applicable to the invention is not particularly limited. As far as the above tissue has been crushed, the form may be pellet-like or in a form dissolved or dispersed in an aqueous solution.

In order to suppress the degradation of nucleic acids contained in the target analyte of the invention to the minimum, it is desirable to apply a collected analyte to the invention in a fresh state without leaving it on standing as far as possible. In the case that it has to be unavoidably stored, it may be stored in a chilled or frozen state. For example, in the case of a blood analyte, the one even stored in a chilled state for about 1 week can be applied to the invention.

The invention is particularly suitable for the extraction of nucleic acids from a blood analyte and the blood analyte may be usual peripheral blood, arterial blood, venous blood, or material component (hemocytes) collected after the bloods have been subjected to centrifugation or the like treatment, buffy coat (inflammatory crust of blood, leukocyte fraction). When whole blood or material components of blood is targeted, steps of the following hemolysis operation and subsequent leukocyte-separating operation are required. In the invention, the kind of blood collecting tube is not particularly limited and any of a heparinized blood collecting tube, an EDTA blood collecting tube, a citric acid blood collecting tube, and the like may be employed. Furthermore, with regard to the blood amount that can be treated in the invention, there is no factor particularly limiting the amount, and the invention can cope with a very wide range of blood amount, e.g., from several µL to several hundred mL.

In the operations of cell lysis and nucleic acid adsorption (and elution), the temperature and period of time are desirably determined in accordance with the predetermined conditions in view of the prevention of unnecessary side reaction and injury of leukocytes or nucleic acids. The following will describe each step of the invention.

### Step for hemolysis and leukocyte separation

The amount of nucleic acids in blood depends on the number of leukocyte cells. In hemolysis, erythrocytes having no nucleus but present in the blood in a large amount (i.e., hemolysis) are destructed, and leukocytes can be separated by centrifugation. The method for hemolysis is not particularly limited and includes a method of utilizing osmotic pressure such as isotonic or hypotonic one, a physical destruction method, and a heat-shock method by a rapid heating. In the invention, a method wherein the heat-shock method is combined is particularly suitable.

A method wherein a hemolytic agent is allowed to act on a blood sample is preferable. Known hemolytic agents include ammonium chloride, ammonium oxalate, and saponin. Of these, those containing at least from 0.01 to 0.5 M ammonium chloride are preferred as hemolytic agents exhibiting a remarkable hemolytic effect under a mild condition. In addition, there may be added, if necessary, a buffer solution for controlling pH, such as Tris buffer solution or phosphate buffer solution; a salt for controlling osmotic pressure, such as sodium chloride or potassium chloride; a magnesium salt for preventing the destruction of leukocytes; a chelating agent for inhibiting DNA degradation, such as EDTA; or a sugar such as saccharose, or the like. In this regard, when a buffer solution is used in combination, the concentration may be optionally selected from the range of several mM to several hundred mM.

The above hemolytic agent is usually used in an amount of preferably 0.1 to 30 equivalent volumes, more preferably 2 to 10 equivalent volumes based on 1 volume of the blood analyte separated into blood cells and blood plasma or serum by centrifugation in the case of whole blood. When the amount used is less than 0.1 volume, the hemolytic effect is decreased and hence the effectiveness is poor. Contrary, when the amount exceeds 30 equivalent volumes, the operation volume becomes large and hence a total treating efficiency including the efficiency of the centrifugal operation remarkably decreases. For an effective dissolution of erythrocytes, it is desirable to use a hemolytic agent containing 0.01 to 0.5M ammonium chloride heated at 30 to 85°C, preferably 40 to 70°C. At a temperature of less than 30°C, a hemolytic effect is low and at an elevated temperature of more than 85°C, the amount of denatured proteins in blood rapidly increases and the proteins precipitate together with target leukocytes at centrifugation, so that a tendency of decreasing purity of the leukocytes becomes remarkable. When a hemolytic operation is carried out under the above conditions, only erythrocytes are completely destructed and leukocytes can be recovered without any damage.

In order to separate leukocytes from the above hemolyzed sample, centrifugation generally used is suitable. The centrifugal conditions such as number of revolution, time, and temperature may be optionally selected depending on a sample state and the like.

### Step for cell lysis

As a lytic operation of leukocytes, known methods for degrading cell membranes, nuclear membranes, proteins, and the like to release nucleic acids from cell nuclei into a lysis solution can be employed.

The methods therefor include methods of destruction of cell membranes utilizing osmotic pressure, shear stress, freeze-crush, mechanical force by means of a grinding agent, or the like, ultrasonic methods utilizing physical energy, chemical or biochemical methods utilizing various surfactants, denaturing agent, or enzymes, or combined procedures thereof.

A preferable operation in the method of the invention is to subject leukocytes to an enzymatic digestion for obtaining cell contents containing target nucleic acids from the separated leukocytes. The enzymatic digestion is carried out by adding a lysis solution containing a cell component-degrading enzyme and a surfactant to a sample containing leukocytes.

### Lysis solution:

It is preferable that a cell-lysing solution, i.e., a lysis solution contains at least one kind of cell component-degrading enzyme and each enzyme concentration is from 0.01 to 50 mg/ml. The enzyme concentration herein is a concentration in the case that a reagent having an enzyme purity of 80% or more is used.

In the invention, the cell component-degrading enzyme means at least one enzyme selected from the group consisting of amylase, lipase, protease, and nuclease. Moreover, when the enzyme concentration is 0.01 mg/ml or less, efficiency of degrading components other than nucleic acids contained in cells becomes extremely low, which is not preferable. When the concentration becomes 50 mg/ml or more, a possibility that the enzyme itself is contaminated in recovered nucleic acids becomes high, which is not preferable.

The amylase herein is an enzyme belonging to 3.2 in accordance with the Enzyme Classification, which decomposes glycosyl compounds. Specifically, α-amylase, β-amylase, glucoamylase, isoamylase, and the like are exemplified. These enzymes may be used singly or in combination. Lipase is an esterase belonging to 3.1 in accordance with the Enzyme Classification.

Protease belongs to 3.4 in accordance with the Enzyme Classification and is a generic name of enzymes specifically hydrolyzing peptide bonds, which usually includes proteinase, peptide hydronase, peptidase, Pronase, and the like. The incorporation of such a proteolytic enzyme enables the increase of degree of separation between nucleic acids and proteins. Specific examples of protease include trypsin, chymotrypsin, pepsin, protease K, Pronase, papain, and analogs and subtypes thereof. These enzymes may be used singly or in combination.

Nuclease is classified into 3.1 according to the Emzyme Classification and is a generic name of an enzyme group hydrolyzing polynucleotide chains of DNA and RNA. In the method according to the invention, both of DNA and RNA can be extracted. Extraction and separation of these nucleic acids can be accelerated by using an RNA nuclease in the case that the nucleic acid to be recovered is DNA and by using a DNA nuclease in the case that RNA is recovered.

These cell component-degrading enzymes may be those extracted from organisms or enzymes prepared by recombinant DNA techniques. Moreover, most of these enzymes are commercially available.

The incorporation of these cell component-degrading enzymes enables the degradation of the components other than target nucleic acids by enzymatic reactions, so that it is possible to separate highly pure nucleic acids. The fragmentation of the components other than the nucleic acids amplifies the solubility in an organic solvent. Moreover, even when the components other than the nucleic acids precipitate, unlike the target nucleic acids that are giant molecules, the components form minute agglomerates and hence do not adhere, deposit, or adsorb onto the solid-phase carrier of the invention, so that they can be easily removed by solid-liquid separation and washing.

On the other hand, since the amount of the enzyme added is very small, the enzyme component added is not strongly adsorbed onto the solid-phase surface because the solid-phase surface is blocked with the cell components. The enzyme is easily removed by usual solid-liquid separation and washing, and is released into a nucleic acid solution, so that it is not extracted together with target nucleic acids.

As the surfactant for use in the above lysis solution, there may be mentioned anionic, cationic, and nonionic surfactants. Of these, in view of the efficiency of destructing leukocyte membranes and the acceleration of efficiency of successive enzymatic reaction, it is desirable to use an anionic surfactant. Specifically, preferred are sodium dodecyl sulfate, sodium laurate, sodium dodecylbenzenesulfonate, sodium sulfate, sodium higher alcohol sulfate, ammonium lauryl sulfate, and the like. These surfactants may be used singly or in combination. The concentration of the surfactant in the lysis solution is preferably from 0.01 to 15% (w/v), more preferably from 0.25 to 10% (w/v).

In addition, a buffer solution for controlling pH (pH 5 to 9), such as Tris buffer solution, a phosphate buffer solution, or a citrate buffer solution, and a salt such as sodium chloride, potassium chloride, lithium chloride, sodium acetate may be added, if necessary. In the case that a buffer solution is used in combination, the concentration may be optionally selected in the rage of several mM to several hundred mM.

### Lysis operation of cells by enzymatic digestion:

In the invention, the above two or more kinds of enzymes may coexist in the lysis solution and may be added at the same time to treat leukocytes or they may be added sequentially to treat leukocytes. When two or more enzymes are used, it is preferable to adjust the concentration of each enzyme in the rage of 0.01 to 50 mg/ml, preferably 0.1 to 10 mg/ml. At the use of these enzymes, in order to enhance the enzymatic activity to the maximum, the buffer solution, salt, ion species, and surfactant are suitably adjusted depending on the kinds of enzymes used, as a matter of course.

In the invention, the enzymatic treatment may be carried out under a heated condition of 30 to 85°C, 7 preferably 40 to 60°C for 0.1 to 10 hours. For the analyte containing particularly many leukocytes, a stronger stirring is preferable in order to lower the viscosity of the reaction system. In the invention, advantageously, a stronger stirring than the stirring obtained by usual vortex can be obtained by the incorporation of the solid-phase carrier. Moreover, the case that the temperature at the enzymatic treatment is less than 30°C is disadvantageous because the time required for the treatment is unnecessarily prolonged. When the temperature exceeds 85°C, degeneration of the enzymes tends to occur, so that the case is not preferable.

### Adsorption step on solid-phase carrier

Usually, the sample containing cell content, which has been subjected to the above cell-lysing treatment is brought, into contact with a solution containing a water-soluble organic solvent containing a solid-phase carrier disclosed below, so that nucleic acids are adsorbed from the sample onto the solid-phase carrier, and thereby the nucleic acids are separated from the other high-molecular weight contaminants.

### Solid-phase carrier:

The composition of the solid-phase carrier of the invention is not particularly limited. It may be fine particles composed of a commercially available organic system, inorganic system, or organic and inorganic composite system or fine particles composed of glass, a ceramic, a metal or metal oxide, or a composite thereof.

The shape of the solid-phase carrier is not particularly limited. Usually, particles are preferred. Examples of the shape of the particles include a sphere, oval sphere, cone, cube, and cuboid. Of these, a carrier of spherical particles is preferable because of easy production and easy rotation or stirring of the solid-phase carrier in use. With regard to the carrier having a sphere shape or a shape other than a sphere, the average particle size or the stokes diameter is in the range of 0.01 to 1000 µm, more preferably in the range of 0.1 to 100 µm. When the diameter is less than 0.01 µm, it takes a lot of time to recover the particles and hence the case is not preferable. Moreover, when the diameter exceeds 1000 µm, the performance tends to be influenced by factors other than the particle size, e.g., conditions of the particle surface.

In this regard, the "stokes diameter" in the invention means an effective diameter in the case that the solid-phase carrier is in the form of particles. With regard to the size of the solid-phase carrier having a shape other than a sphere, a diameter determined in accordance with Stokes' law is regarded as a diameter of a spherical carrier having an identical stokes diameter.

The stokes diameter of the solid-phase carrier having a shape other than a sphere can be determined as follows. That is, the solid-phase carrier having a shape other than a sphere is dispersed in an aqueous solution and subjected to centrifugal sedimentation to determine the sedimentation rate based on a Stokes' equation. The sedimentation rate of a sphere shape carrier of the same material having a known average particle size is also determined by the same way. The average particle size of the sphere shape carrier showing the same sedimentation rate is regarded as a stokes diameter of the solid-phase carrier having a shape other than a sphere.

in addition to the case that the whole carrier including the surface is composed of the same material, the above solid-phase carrier may be a hybrid composed of a plurality of materials as occasion demands. In this case, the material composing the surface of the solid-phase carrier is selected from the group consisting of synthetic polymers. More specifically, in order to cope with an automated equipment, it is possible to introduce a magnetically responsive material such as iron oxide or chromium oxide into the particles.

Specifically, these solid-phase carriers can be prepared through organic synthetic reactions. For example, the carrier is a polymer (hereinafter referred to as "specific polymer") of one monomer selected from the group consisting of aromatic vinyl compounds, polyacrylate esters, and polymethacrylate esters, and if necessary, a carboxyl group can be introduced into the surface of the organic polymer particles by copolymerization with an α, β-unsaturated carboxylic acid monomer.

A material constituting the above water-insoluble solid-phase carrier may be any of water-insoluble materials. The "water-insoluble" material specifically means a solid-phase insoluble in water and an aqueous solution containing any other water soluble composition. Specifically, the material includes an inorganic compound, a metal, a metal oxide, an organic compound, or a composite material obtained by combining them.

A specific material to be used as the solid-phase carrier is a synthetic organic polymer such as a polyacrylate or polymethyl methacrylate.

### Water-soluble organic solvent:

As a water-soluble organic solvent for use in the invention, a hydroxyl group-containing solvent soluble in water is mentioned and an alcohol is particularly preferred. Specifically, as the alcohol, at least one compound is selected from the group consisting of butanol, 2-butanol, pentanol, 2-pentanol, methanol, ethanol, propanol, and isopropanol. The water-soluble organic solvents may be used singly or as a mixture of two or more of them. Of these, particularly preferred are ethanol and isopropanol.

When these water-soluble organic solvents are used, the concentration of the water-soluble organic solvent at nucleic acid extraction is from 25 to 100% by volume, preferably from 40 to 100% by volume. Usually, the nucleic acids released from cells or a host are solely precipitated and adsorbed on the surface of the solid-phase carrier at the above concentration of the organic solvent.

If necessary, a salt, a water-soluble polymer, a polysaccharide, and/or a surfactant may be added prior to or simultaneously with the addition of the above water-soluble organic solvent. Examples of the additives include sodium salts, potassium salts, magnesium salts, polyvinyl alcohol, agarose, dextran, dextran sulfate, sodium dodecylsulfonate, and the like. The addition of these additives frequently results in a more highly efficient capture of nucleic acids onto the solid-phase carrier and easy re-dissolution thereof.

The concentration of the salt to be added in the solvent solution is preferably from 0.1 to 50 mM, more preferably from 0.5 to 10 mM. When the salt concentration is less than 0.1 mM, the nucleic acid-precipitating effect by the added salt is small and hence the case is not preferable. Contrary, the salt concentration exceeding 50 mM causes precipitation of the substances other than nucleic acids, such as hydrophilic proteins, so that the salting-out effect of only nucleic acids is reduced. Similarly, the concentration of the water-soluble polymer or polysaccharide in the solvent solution is from 0.0001 to 10% (w/v) and the concentration of the surfactant is preferably 0.01 to 15% (w/v), particularly 0.05 to 0.5% (w/v). When the surfactant concentration is less than 0.01% (w/v), a micelle effect of hydrophobic proteins is weak, and when it exceeds 15% (w/v), precipitation of the surfactant is invited, so that both cases are not preferable.

### Adsorption operation:

In the invention, nucleic acids are adsorbed on the solid phase of the solid-phase carrier by bringing a sample containing the nucleic acids into contact with the solid-phase carrier in the presence of the water-soluble organic solvent.

At that time, the order for contacting the lysis solution, the solid-phase carrier, and the water-soluble organic solvent is not limited.

That is, any of (a) a procedure wherein the solid-phase carrier is added simultaneously at the cell lysis, (b) a procedure wherein the solid-phase carrier is added after the cell lysis, and (c) the solid-phase carrier is first brought into contact with the sample directly and then the cell lysis is carried out is applicable.

However, in the case (c) that the solid-phase carrier is first brought into contact with the sample, it is preferable to add the lysis solution immediately after the contact of the solid-phase carrier with the sample.

Among these procedures, particularly preferred is (b) a procedure wherein the solid-phase carrier is added after the cell lysis. In this case, the solid-phase carrier may be added together with the water-soluble organic solvent or separately.

In the invention, although the amount of the solid-phase carrier varies depending on the particle size, the carrier is preferably used in an amount of about 0.01 to 100 mg in terms of the dried particle weight per 1 ml of the analyte sample.

The period of time required for adsorption of nucleic acids on the solid-phase carrier is usually from 0.1 to 20 minutes, preferably from 3 to 15 minutes after mixing of the water-soluble organic solvent, the sample containing nucleic acids and the solid-phase carrier. In particular, in the case of extracting a long nucleic acid such as genome DNA, it is preferable to lengthen the period of the adsorption step. Moreover, the adsorption temperature is from 0 to 60°C, preferably from 4 to 25°C.

In the adsorption step, when the reaction mixture is stirred, the rate is usually from 0.5 to 20 revolutions/minute, preferably from 1 to 5 revolutions/minute.

### Washing and elution step

After the step of adsorption of nucleic acids on the solid-phase carrier is completed, the solid-phase carrier is separated from the reaction mixture and the solid-phase carrier separated is washed. Moreover, before the liquid washing of the nucleic acids, the remaining protein components may be dissolved and removed by immersing the carrier in a protein-dissolving liquid such as guanidine hydrochloride, guanidine thiocyanate, or urea. The washing liquid may be any of the same solvent as the water-soluble organic solvent used in the adsorption step and the other water-soluble solvent as far as it does not elute the nucleic acids bound to the solid-phase carrier. Moreover, if necessary, the concentration of the water-soluble organic solvent may be lowered as far as the adsorption of the nucleic acids on the solid-phase carrier can be maintained.

The nucleic acids adsorbed on the solid-phase carrier may be used in the next step after the above washing and successive drying. The method for drying is not limited, and drying under reduced pressure, drying under heating, drying under air circulation, or the like is employed. In particular, air drying is desirable as the drying method. The drying temperature is usually from room temperature to 60°C, and the period for drying is preferably from 5 minutes to 20 minutes.

The nucleic acid-adsorbed solid-phase carrier thus obtained may be subjected to the following steps as it is depending on intended purposes, for example, various nucleic acid analyses such as amplification by PCR method or treatment with a restriction enzyme. Alternatively, the nucleic acids adsorbed on the solid-phase carrier may be eluted into a liquid layer by adding sterile distilled water or Tris hydrochloride/EDTA buffer solution (TE) to the dried solid-phase carrier and stirring the resulting mixture.

For eluting the nucleic acids from the solid-phase carrier, a solvent or buffer solution having a low ionic strength is allowed to act on the solid-phase carrier and, if necessary, the mixture is heated. The temperature for elution of the nucleic acids is preferably from 50 to 90°C, more preferably from 70 to 80°C. Examples of such an eluent include water, Tris hydrochloride buffer solution, phosphate buffer solution, and the like. The nucleic acids eluted are recovered through removal of the carrier by an operation such as centrifugation, filtration, or decantation.

### Embodiments of nucleic acid separation method

The embodiments according to the invention are not particularly limited and various embodiments are possible depending on samples and purposes.

When nucleic acids are extracted and purified from blood samples capable of being extremely easily collected from test subjects, the invention is particularly applicable for the purpose of utilizing data on nucleic acids in a clinical field. According to the method of the invention, both of DNA and RNA can be separated and the nucleic acids obtained require no further purification and can be subjected to PCR method and various analyses as they are.

The method for separating nucleic acids of the invention can be also carried out throughout all the procedures in a single reaction vessel, which may result in the following advantage.
(1) At the first stage of the method, since the nucleic acids released from a sample containing the nucleic acids bind onto at least most of the solid phase during the successive purification step, a risk of contamination or exposure can be extremely lowered. With regard to the exposure to pathogenic bacteria, a risk to a human body involved in the treatment of an analyte having a possibility of infection with a pathogenic bacterium or virus is almost limited to the first stage of the separation step wherein the sample is introduced into a reaction vessel. In the early stage of the treatment, the virus or bacterium is efficiently inactivated. As another kind of contamination, there is a problem of mutual contamination in the case of treating a large number of analytes. The risk of contamination can be avoided or reduced to the minimum by carrying out the method in a single reaction vessel.
(2) It is convenient for realizing automation of the treatment.

The automation of the extraction and preparation of nucleic acids enables a rapid treatment of a large number of analytes without requiring skilled operation and advanced knowledge. Therefore, the automation of nucleic acid preparation has a wide range of applications and uses in individual fields requiring nucleic acid analysis including genetic engineering and genetic diagnosis and hence has an extremely important significance.

The method of the invention is extremely suitable for the purpose of rapid mechanical separation of nucleic acids from a large number of samples, i.e., automatic treatment, based on the above-mentioned characteristic feature thereof. This is because the method of the invention requires no two-phase extraction of water/phenol and also basically does not require centrifugal operation except for the step of separating leukocytes, so that there exists no particular barrier for mechanization.

### Nucleic acid separation Reagent (kit):

The invention also provides a reagent kit for separation and purification of nucleic acids from an analyte sample containing nucleated cells. The reagent of the invention comprise at least a lysis solution, a water-insoluble solid-phase carrier, and a water-soluble organic solvent.

The individual components constituting the kit may be arranged separately or may be combined unless there arises any trouble.

Furthermore, if necessary, the kit may contain an enzyme solution, a hemolytic agent, a washing liquid, an eluent, an auxiliary, a special container, and other necessary accessories.

With regard to the "lysis solution", "water-insoluble solid-phase carrier", and "water-soluble organic solvent", those described in the above are all suitably employed.

As the "washing liquid" and "eluent", the same solvent as the water-soluble organic solvent used in the adsorption step or other water-soluble solvent, a phosphate buffer solution, Tris hydrochloride buffer solution, and the like may be exemplified. The both may be the same type of buffer solution and the action for washing or elution can be realized by changing the liquid nature of the buffer solution, specifically changing ionic strength, pH, or kind of salts contained.

The following will describe the invention specifically with reference to Examples but the invention is not limited to these Examples.

### Reference Example 1

To 1 ml of whole blood containing heparin anticoagulant in a 12 ml volume test tube was added 4 ml of a hemolytic agent, i.e., a solution containing 0.15 M NH₄Cl, 20 mM MgCl₂, and 20 mM CaCl₂ in 50mM Tris-hydrochloride buffer solution (pH 7.6), heated to 70°C. After stirring upward and downward, the mixture was left on standing for 5 minutes and then centrifuged (3000 rpm × 5 minutes) and the supernatant was removed.

To the pellet present at the bottom of the test tube was added 0.1 ml of a 200 mM potassium chloride solution containing tripsin (5 mg/ml), RNA nuclease (2 mg/ml), lipase (2 mg/ml), amylase (1 mg/ml), 0.5% (w/v) sodium laurate, followed by 10 minutes of heating at 50°C. After completion of the reaction, 10 µl of a dispersion (10% by weight) of magnetic particles having an average diameter of 20 µm whose surface was constituted by polystyrene and whose inner part by iron oxide and polystyrene was added, followed by subjecting to vortex. Subsequently, 2 ml of a 50% by volume IPA/50% by volume of ethanol mixed solution was added thereto and the mixture was stirred with a Vortex mixer to adsorb DNA on the particles. Then, a magnet was brought near to the side of the test tube to fix the particles and then the supernatant was removed. This washing operation was repeated twice. Subsequently, after washing twice with 1 ml of 20 mM Tris buffer solution (pH 7.6), 0.5 ml of TE was added and the resulting mixture was heated at 60°C with stirring for 10 minutes and then the above polystyrene magnetic beads were magnetically separated to recover a liquid layer part.

DNA contained in the above recovered supernatant was quantitatively determined by measurement of absorbance. The amount of recovered DNA was calculated from UV absorbance at 260 nm (A260) and the purity was confirmed by the ratios of absorbance (A260/A280, A260/A230) to confirm that both of them were found to be 1.9 or more. Moreover, the length of DNA was investigated by a pulse electrophoresis on 1% by weight agarose gel. As a result, DNA was distributed in the region of 100K to 200K. A digestion result after the recovered DNA was subjected to an enzymatic treatment with 0.2 unit of restriction enzymes HindIII and EcoRI was confirmed by an electrophoresis. As a result, it was confirmed that the DNA was the same level as the DNA recovered with phenol-chloroform. Furthermore, amplification of a globulin gene was carried out using 1 fg DNA as a template to confirm that a PCR reaction could be carried out without trouble.

Excluding the time for preparation of the reagent, the period of time required for this experiment was about 1 hour when the experiment was carried out with 10 tubes at the same time.

### Reference Example 2

A similar operation to Example 1 was carried out with the exception that pepsin having the same concentration as the case of trypsin was used instead of the enzyme trypsin used in Example 1.

### Reference Example 3

A similar operation to Example 1 was carried out with the exception that proteinase K was used at the same concentration instead of the enzyme trypsin used in Example 1.

### Reference Example 4

A similar operation to Example 1 was carried out with the exception that peptidase having the same concentration as the case of trypsin was used instead of the enzyme trypsin used in Example 1.

### Reference Example 5

A similar operation to Example 1 was carried out with the exception that polystyrene crosslinked particles having a particle size of 30 µm was used instead of the solid-phase carrier composed of polystyrene/iron oxide used in Example 1.

### Reference Example 6

To a cell lysis solution after enzymatic treatment in a similar manner to Example 1 was added the same volume of 5 M guanidine hydrochloride. After stirring with shaking, the mixture was left on standing at room temperature for 5 minutes. Thereafter, in a similar manner to Example 1, 10 µl of a dispersion (10% by weight) of magnetic particles having an average diameter of 20 µm whose surface was constituted by polystyrene and whose inner part by iron oxide and polystyrene was added, followed by subjecting to vortex. The successive operations were carried out as in Example 1.

### Reference Example 7

At the treatment of pellet of cell components in Example 1 with tripsin (5 mg/ml), RNA nuclease (2 mg/ml), lipase (2 mg/ml), and amylase (1 mg/ml), 0.1 ml of 200 mM potassium chloride solution containing 0.5% (w/v) sodium laurate and 20 µl of 0.3% dextran (molecular weight 50,000) were added and the mixture was heated at 50°C for 10 minutes. The successive operations were carried out as in Example 1.

### Example 8

To 10 ml of blood collected from an EDTA blood-collecting tube was added 60 ml of 0.15 M NH₄Cl heated to 40°C. After stirring upward and downward, the mixture was centrifuged at 3000 rpm x 5 minutes and the supernatant was removed. To the pellet was added 0.5 ml of 200 mM potassium chloride solution containing peptidase (5 mg/ml), lipase (2 mg/ml), 0.5% (w/v) sodium dodecyl sulfate, followed by 0.5 hour of heating at 50°C. After completion of the reaction, 5 µl of a dispersion (particle solid mass 5%) of polyacrylate resin particles having a particle size of 5 µm was added and subjected to vortex. Then, 1 ml of ethanol was added thereto and the test tube was stirred three times upward and downward. Thereafter, the particles were fixed to the test tube wall magnetically and the liquid layer was removed. Then, the particles were washed twice with 50% isopropyl alcohol (IPA) aqueous solution and finally magnetically separated particle pellets were washed once with 1 ml of 20 mM Tris buffer solution (pH 7.6) and then added to 0.5 ml of TE, followed by 30 minutes of heating at 70°C under stirring. Finally, the particle dispersion was subjected to solid-liquid separation with a magnet to recover the supernatant.

### Comparative Example 1

As a reference experiment, the leukocyte precipitate obtained in Example 1 was extracted by the phenol-chloroform method. The extraction method was carried out in accordance with "Molecular Cloning" (J. Sambrook, 1982, CSH publishing). Excluding the time for preparation of the reagent, the period required for this reference experiment was about 3 hour when the experiment was carried out with 10 tubes at the same time.

The results of Examples and Comparative Example are shown in the following Table 1.

**Table 1**

| | Recovered DNA concentration µg/ml | Amount of recovered DNA µg | A260/ A280 | A260/ A230 | DNA length | HindIII, EcoRI digestion | Globulin PCR |
|---|---|---|---|---|---|---|---|
| Example 1* | 168 | 34 | 1.95 | 2.03 | 150K | No trouble | No trouble |
| Example 2* | 160 | 32 | 1.98 | 2.01 | 150K | No trouble | No trouble |
| Example 3* | 185 | 37 | 2.05 | 2.06 | 150K | No trouble | No trouble |
| Example 4* | 177 | 35 | 2.04 | 1.99 | 150K | No trouble | No trouble |
| Example 5* | 184 | 37 | 1.98 | 1.97 | 150K | No trouble | No trouble |
| Example 6* | 199 | 40 | 1.99 | 1.99 | 150K | No trouble | No trouble |
| Example 7* | 184 | 37 | 2.08 | 2.04 | 150K | No trouble | No trouble |
| Example 8 | 183 | 365 | 1.97 | 1.98 | 150K | No trouble | No trouble |
| Comparative Example 1 | 120 | 24 | 1.99 | 2.01 | 150K | No trouble | No trouble |

| | | | | | | | |
|---|---|---|---|---|---|---|---|
| *Reference Example | | | | | | | |

By using the method for extracting nucleic acids of the invention and the reagent thereof, nucleic acids can be purified from blood etc. in a large amount and a high purity, conveniently within a short period at a low cost. Also, the invention establishes a method that uses no toxic or corrosive solvent and thus is not harmful to working environment and workers. Therefore, the method is widely applicable to the fields of gene engineering, genetic diagnosis, genetic therapy, genome chemistry, genomic drug development, and the like. Moreover, the method is capable of automation of the treatment.

While the invention has been described in detail and with reference to specific embodiments thereof, it will be apparent to one skilled in the art that various changes and modifications can be made therein without departing from the scope thereof.

This application is based on Japanese patent application No. 2002-220099 filed July 29, 2002, the entire contents thereof being hereby incorporated by reference.

A method for purifying nucleic acids wherein objective nucleic acids are separated through solid-liquid separation by deposition of the nucleic acids onto a solid-phase carrier, via enzymatic treatment of a cell lysis solution, from a sample containing nucleated cells and the like, as well as a kit for carrying out the method.

## Claims

1. A method for separating nucleic acids, wherein nucleic acids are separated and purified from a sample containing nucleated cells, comprising:
1) a step of bringing the sample containing nucleated cells into contact with
a lysis solution containing at least a cellular component-degrading enzyme, a surfactant and a water-insoluble solid-phase carrier having an average particle size of 0.01 to 1000 µm and having a surface composed of a material selected from the group consisting of polyacrylates and polymethyl methacrylates
in the presence of a water-soluble organic solvent, thereby adsorbing and binding nucleic acids released from the nucleated cells onto the surface of said water-insoluble solid-phase carrier, and
2) a step of separating the solid-phase carrier from the sample.

2. The method for separating nucleic acids according to claim 1, wherein the cellular component-degrading enzyme is at least one enzyme selected from the group consisting of amylase, lipase, protease, and nuclease.

3. The method for separating nucleic acids according to claim 1, wherein the surfactant is an anionic surfactant.

4. The method for separating nucleic acids according to claim 1, which further comprises 4) a step of washing the separated solid-phase carrier.

5. The method for separating nucleic acids according to claim 1, which further comprises 5) a step of eluting the nucleic acids adsorbed onto the solid-phase carrier.

6. A nucleic acid-extracting reagent kit for separating and purifying nucleic acids from a sample containing nucleated cells, comprising at least a cellular component-degrading enzyme, a water-insoluble solid-phase carrier as defined in claim 1, a surfactant and a water-soluble organic solvent.

## Patentansprüche

1. Verfahren zur Abtrennung von Nukleinsäuren, wobei die Nukleinsäuren abgetrennt und gereinigt werden aus einer Zellen mit Zellkern enthaltenden Probe, einschließend:
1) ein Schritt, die Zellen mit Zellkern enthaltende Probe in Kontakt zu bringen mit einer Lyse-Lösung, enthaltend zumindest ein Zellbestandteil-zersetzendes Enzym, einen oberflächenaktiven Stoff und einen wasserunlöslichen Festphasenträger mit einer durchschnittlichen Partikelgröße von 0,01 bis 1000 µm und mit einer Oberfläche, zusammengesetzt aus einem Material ausgewählt aus der Gruppe, bestehend aus Polyacrylaten und Polymehtyl-Methacrylaten
in der Gegenwart eines wasserlöslichen organischen Lösungsmittels, wodurch aus den Zellen mit Zellkern freigegebene Nukleinsäuren auf die Oberfläche des wasserunlöslichen Festphasenträgers adsorbiert und gebunden werden, und
2) ein Schritt, den Festphasenträger von der Probe abzutrennen.

2. Verfahren zur Abtrennung von Nukleinsäuren nach Anspruch 1, wobei das Zellbestandteil-zersetzende Enzym zumindest ein Enzym ist, ausgewählt aus der Gruppe bestehend aus Amylase, Lipase, Protease, und Nuclease.

3. Verfahren zur Abtrennung von Nukleinsäuren nach Anspruch 1, wobei der oberflächenaktive Stoff ein anionischer oberflächenaktiver Stoff ist.

4. Verfahren zur Abtrennung von Nukleinsäuren nach Anspruch 1, welches ferner einschließt 4) einen Schritt des Waschens des abgetrennten Festphasenträgers.

5. Verfahren zur Abtrennung von Nukleinsäuren nach Anspruch 1, welches ferner einschließt 5) einen Schritt des Eluierens der auf dem Festphasenträger adsorbierten Nukleinsäuren.

6. Nukleinsäure-extrahierender Reagenziensatz zur Abtrennung und Reinigung von Nukleinsäuren aus einer Zellen mit Zellkern enthaltenden Probe, zumindest einschließend ein Zellbestandteil-zersetzendes Enzym, einen wasserunlöslichen Festphasenträger nach Anspruch 1, einen oberflächenaktiven Stoff und ein wasserlösliches organisches Lösungsmittel.

## Revendications

1. Procédé pour séparer des acides nucléiques où les acides nucléiques sont séparés et purifiés à partir d'un échantillon contenant des cellules nucléées, comprenant :
1) une étape de mise en contact de l'échantillon contenant des cellules nucléées avec
une solution de lyse contenant au moins une enzyme dégradant des composants cellulaires, un tensioactif et un support en phase solide insoluble dans l'eau ayant une taille de particule moyenne de 0,01 à 1 000 µm et ayant une surface composée d'une substance choisie dans le groupe consistant en les polyacrylates et les poly(méthacrylates de méthyle),
en présence d'un solvant organique soluble dans l'eau, pour ainsi adsorber et lier les acides nucléiques libérés par les cellules nucléées sur la surface dudit support en phase solide insoluble dans l'eau, et
2) une étape de séparation du support en phase solide d'avec l'échantillon.

2. Procédé pour séparer des acides nucléiques selon la revendication 1, où l'enzyme dégradant des composants cellulaires est au moins une enzyme choisie dans le groupe consistant en une amylase, une lipase, une protéase et une nucléase.

3. Procédé pour séparer des acides nucléiques selon la revendication 1, où le tensioactif est un tensioactif anionique.

4. Procédé pour séparer des acides nucléiques selon la revendication 1 qui comprend en outre 4) une étape de lavage du support en phase solide séparé.

5. Procédé pour séparer des acides nucléiques selon la revendication 1 qui comprend en outre 5) une étape d'élution des acides nucléiques adsorbés sur le support en phase solide.

6. Kit de réactifs d'extraction d'acides nucléiques pour séparer et purifier des acides nucléiques à partir d'un échantillon contenant des cellules nucléées, comprenant au moins une enzyme dégradant des composants cellulaires, un support en phase solide insoluble dans l'eau selon la revendication 1, un tensioactif et un solvant organique soluble dans l'eau.
